(19) Europäisches Patentamt
European Patent Office
Office européen des brevets

(11) Veröffentlichungsnummer: **0 087 056**
**B1**

(12) # EUROPÄISCHE PATENTSCHRIFT

(45) Veröffentlichungstag der Patentschrift: **15.01.86**

(51) Int. Cl.[4]: **C 07 C 121/75**, C 09 K 19/12, C 09 K 19/32, G 02 F 1/13

(21) Anmeldenummer: **83101167.1**

(22) Anmeldetag: **08.02.83**

(54) **Benzonitrile.**

(30) Priorität: **18.02.82 DE 3205766**

(43) Veröffentlichungstag der Anmeldung: **31.08.83 Patentblatt 83/35**

(45) Bekanntmachung des Hinweises auf die Patenterteilung: **15.01.86 Patentblatt 86/3**

(84) Benannte Vertragsstaaten: **CH DE FR GB LI**

(56) Entgegenhaltungen:
**EP-A-0 006 188**
**EP-A-0 023 728**
**DE-A-2 636 684**
**US-A-4 368 135**

(73) Patentinhaber: **Merck Patent Gesellschaft mit beschränkter Haftung, Frankfurter Strasse 250, D-6100 Darmstadt (DE)**

(72) Erfinder: **Eidenschink, Rudolf, Dr., Erlenweg 17, D-6110 Dieburg (DE)**
Erfinder: **Eichler, Jürgen, Dr., Ringstrasse 28, D-6127 Breuberg (DE)**
Erfinder: **Weber, Georg, Wilhelm- Leuschner-Strasse 38, D-6106 Erzhausen (DE)**

EP 0 087 056 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft neue Benzonitrile der Formel I

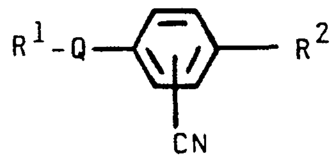

I

worin

$R^1$ und $R^2$ jeweils Alkylgruppen mit 1-8 C-Atomen, einer dieser Reste auch eine Alkoxygruppe mit 1-8 C-Atomen und

Q einen oder zwei Reste, ausgewählt aus der Gruppe bestehend aus 1,4-Phenylen und/oder 1,4-Bicyclo[2,2,2]octylen bedeuten.

Diese Substanzen können wie ähnliche, z.B. aus der DE-OS 29 33 563 bekannte Verbindungen als Komponenten flüssigkristalliner Dielektrika verwendet werden, insbesondere für Displays, die auf dem Prinzip der dynamischen Streuung beruhen. Gegenüber den in dieser DE-OS allein konkret genannten Estern zeigen die vorliegenden Verbindungen bessere Stabilitäten und stärker negative DKA-Werte.

Für elektrooptische Anzeigeelemente, deren Arbeitsweise auf dem Phänomen der dynamischen Streuung, der Deformation aufgerichteter Phasen (DAP-Effekt) oder der Absorption des Lichtes durch orientierbare dichroitische Farbstoffe beruht, werden flüssigkristalline Dielektrika mit deutlicher negativer dielektrischer Anisotropie (DKA), bei denen die Dielektrizitätskonstante (DK) parallel zur Molekülachse kleiner ist als die DK senkrecht zur Molekülachse, benötigt. Die Schwellenspannung für den Betrieb derartiger Anzeigeelemente ist umso kleiner, je negativer die DKA des flüssigkristallinen Dielektrikums ist. Eine weitere Grundanforderung an derartige Dielektrika ist ein breiter, die Raumtemperatur einschließender Temperaturbereich der nematischen Phase.

Unter den gebräuchlichen flüssigkristallinen Basismaterialien solcher Dielektrika gibt es keine mit ausgeprägt negativer DKA. Um daraus flüssigkristalline Dielektrika mit ausgeprägter negativer DKA herzustellen, ist der Zusatz von flüssigkristallinen Verbindungen mit sehr stark negativer DKA erforderlich. Diese sind gewöhnlich in den flüssigkristallinen Basismaterialien nicht ausreichend löslich, als Ester nicht stabil und/oder bewirken eine unerwünschte Verschiebung des nematischen Temperaturbereiches. Die Löslichkeitsprobleme lassen sich zur Not durch Verwendung von mehreren Verbindungen mit negativen DKA unter Inkaufnahme einer Erhöhung der Viskosität der nematischen Phase und in deren Folge einer unerwünschten Erhöhung der Schaltzeiten der Anzeigeelemente.

Der Erfindung lag die Aufgabe zugrunde, neue stabile, niedrig viskose, flüssigkristalline Basismaterialien für Dielektrika mit deutlich negativer DKA aufzufinden. Diese Aufgabe wurde mit der Bereitstellung der Verbindungen der Formel I gelöst.

Es wurde gefunden, dass die Benzonitrile der Formel I als Komponenten flüssigkristalliner Dielektrika vorzüglich geeignet sind. Insbesondere sind mit ihrer Hilfe stabile flüssigkristalline Phasen mit stark negativer dielektrischer Anisotropie (DKA) herstellbar. Aus der für flüssigkristalline Basismaterialien unüblichen Anordnung der Nitrilgruppen seitlich zur Moleküllängsachse resultiert offenbar die ausgeprägt negative dielektrische Anisotropie, welche Voraussetzung ist für die Verwendung dieser Verbindungen für elektrooptische Anzeigen, die auf dem Phänomen der dynamischen Streuung, der Deformation aufgerichteter Phasen oder der Lichtabsorption durch orientierbare dichroitische Farbstoffe beruhen.

Damit besitzen die Verbindungen der Formel I einen ausserordentlich breiten Anwendungsbereich. In Abhängigkeit von der Auswahl der Substituenten können diese Verbindungen als Basismaterialien dienen, aus denen flüssigkristalline Dielektrika zum überwiegenden Teil zusammengesetzt sind, es können aber auch Verbindungen der Formel I flüssigkristallinen Basismaterialien aus andern Verbindungsklassen zugesetzt werden, um beispielsweise die mittlere DKA eines solchen Dielektrikums zu senken.

Die Verbindungen der Formel I sind in reinem Zustand farblos und bilden flüssigkristalline Mesophasen in für die elektrooptische Verwendung gut verwertbarem Temperaturbereich. Als Benzonitrile sind sie stabil.

Gegenstand der Erfindung sind somit die Benzonitrile der Formel I, Verfahren zu ihrer Herstellung und ihre Verwendung als Komponenten flüssigkristalliner Dielektrika. Gegenstand der Erfindung sind weiterhin flüssigkristalline Dielektrika mit einem Gehalt an mindestens einem Benzonitril der Formel I sowie elektrooptische Anzeigeelemente auf der Basis einer Flüssigkristallzelle, die ein derartiges flüssigkristallines Dielektrikum enthält.

Die erfindungsgemäßen Benzonitrile der Formel I umfassen insbesondere die bevorzugten Phenylbenzonitrile der Formel Ia und Bicyclo[2,2,2]-octylen-phenyl-benzo-nitrile der Formel Ib

$R^1$-Phe-Phe(CN)-$R^2$ Ia

$R^1$-Bi-Phe-Phe(CN)-$R^2$ Ib

worin jeweils Phe einen 1,4-Phenylenrest,

Bi einen Bicyclo[2,2,2]octylenrest und Phe(CN) einen in 2- oder 3- bzw. 5- oder 6-Stellung durch eine Nitrilgruppe substituierten 1,4-Phenylenrest bedeutet.

Weiterhin umschließt die Formel I bevorzugt die Benzonitril-derivate der Formeln Ic bis If:

| | |
|---|---|
| $R^1$-Bi-Phe(CN)-$R^2$ | Ic |
| $R^1$-Phe-Phe-Phe(CN)-$R^2$ | Id |
| $R^1$-Phe-Bi-Phe(CN)-$R^2$ | Ie |
| $R^1$-Bi-Bi-Phe(CN)-$R^2$ | If, |

worin $R^1$, $R^2$, Bi, Phe und Phe (CN) die angegebene Bedeutung haben.

In den Verbindungen der Formel I können die Alkyl- bzw. Alkoxy-reste $R^1$ und $R^2$ im Prinzip geradkettig oder verzweigt sein.

Vorzugsweise sind sie geradkettig und bedeuten demnach bevorzugt Methyl, Ethyl, n-Propyl, n-Butyl, n-Pentyl, n-Hexyl, n-Heptyl, n-Octyl oder Methoxy, Ethoxy, n-Propoxy, n-Butoxy, n-Pentoxy, n-Hexoxy, n-Heptoxy oder n-Octoxy.

Verbindungen der Formel I mit geradkettigen Flügelgruppen $R^1$ und $R^2$ weisen in der Regel höhere Klärpunkte auf als die analogen Verbindungen mit verzweigten Flügelgruppen $R^1$ und $R^2$.

Deswegen sind die Verbindungen der vorliegenden Erfindung gewöhnlich höchstens mit einer verzweigten Flügelgruppe $R^1$ oder $R^2$ ausgerüstet.

Verbindungen der Formel I mit einer verzweigten Flügelgruppe $R^1$ und $R^2$ können gelegentlich wegen einer besseren Löslichkeit in den üblichen flüssigkristallinen Basismaterialien von Bedeutung sein, insbesondere aber als chirale Dotierstoffe, wenn sie durch die Kettenverzweigung optische Aktivität besitzen. Verzweigte Flügelgruppen $R^1$ oder $R^2$ enthalten in der Regel nicht mehr als eine Kettenverzweigung. Bevorzugte verzweigte Reste $R^1$ und $R^2$ sind Isopropyl, 2-Methylpropyl, 2-Methylbutyl, 3-Methylbutyl, 2-Methylpentyl, 3-Methylpentyl, 2-Ethylhexyl, 1-Methylhexyl, 1-Methylheptyl, 2-Ethyl-pentyl, Isopropyloxy, 2-Methylpropyloxy, 2-Methylbutyloxy, 3-Methylbutyloxy, 2-Methylpentyloxy, 3-Methylpentyloxy, 2-Ethylhexyloxy, 1-Methylhexyloxy, 1-Methylheptyloxy. Die Gruppen $R^1$ und $R^2$ enthalten jeweils 1 bis 8, vorzugsweise 3 bis 5 C-Atome. Die Alkylgruppen sind vor den Alkoxygruppen bevorzugt.

Die Verbindungen der Formel I werden nach an sich für ähnliche Verbindungen bekannten Methoden hergestellt, wie sie in der Literatur (z.B. in den Standardwerken wie Houben-Weyl, Methoden der organischen Chemie, Georg Thieme Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können gewünschtenfalls auch in situ gebildet werden, derart, dass man sie aus dem Reaktionsgemisch nicht isoliert, sondern sofort weiter zu den Verbindungen der Formel I umsetzt.

Die Verbindungen der Formel I, worin $R^2$ eine Alkoxygruppe darstellt, werden bevorzugt hergestellt, indem man als Ausgangsmaterial etwa eine bekannte Verbindung der Formel

$R^1$-Q-Phe-$R^2$

durch eine elektrophile Substitution in eine Verbindung der Formel

$R^1$-Q-Phe(X)-$R^2$

worin X ein Halogenatom, Cl, Br oder Jod oder eine Nitrogruppe $NO_2$ bedeutet, umsetzt, den Substituenten X falls er $-NO_2$ bedeutet, durch Reduktion und Diazotierung in eine Diazoniumsalzgruppe umwandelt und schliesslich die Halogen- oder Diazoniumsalz-gruppe durch Umsetzung mit einem Metallcyanid in die Nitrilgruppe überführt. Als Metallcyanide kommen bevorzugt Schwermetallcyanide, vorzugsweise $Cu_2(CN)_2$ in Betracht. Die Umsetzung erfolgt in Wasser oder besser in Gegenwart eines inerten Lösungsmittels wie Dimethylformamid, N-Methyl-pyrrolidon oder Dimethylsulfoxid bei Temperaturen zwischen etwa 0 und 150 Grad Celsius.

Die Verbindungen der Formel werden alternativ erhalten, wenn man ein Amid der Formel

$R^1$-Q-Phe($CO-NH_2$)-$R^2$

oder ein Oxim der Formel

$R^1$-Q-Phe($CH{=}NOH$)-$R^2$

dehydratisiert. Als wasserabspaltende Mittel eignen sich beispielsweise anorganische Säurechloride wie $SOCl_2$, $PCl_3$, $PCl_5$, $SO_2Cl_2$, $COCl_2$, ferner $P_2O_5$, $P_2S_5$, $AlCl_3$ als Doppelverbindung mit NaCl, aromatische Sulfonsäuren und Sulfonsäurehalogenide. Man arbeitet in Gegenwart oder Abwesenheit eines inerten Lösungsmittels, beispielsweise eines aromatischen Kohlenwasserstoffs wie Benzol, Toluol oder Xylol, vorzugsweise bei Temperaturen zwischen etwa 50 und 150 Grad Celsius.

Das Verfahren zur Herstellung der Verbindungen der Formel I ist demnach dadurch gekennzeichnet, dass man eine Verbindung der Formel II

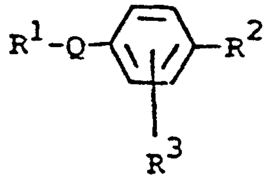

II

worin
$R^3$ Cl, Br, J oder eine Diazoniumsalzgruppe bedeutet und
$R^1$ und $R^2$ die angegebene Bedeutung haben mit einem Metallcyanid umsetzt oder daß man eine Verbindung der Formel III

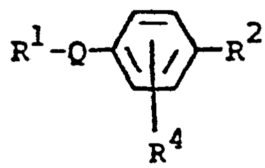

III

worin
$R^4$ $-CONH_2$ oder $-CH{=}NOH$
bedeutet und
$R^1$ und $R^2$ die angegebene Bedeutung haben dehydratisiert.

Die Ausgangsstoffe der Formeln II und III sind grösstenteils neu. Sie können aber nach an sich bekannten Methoden hergestellt werden. Die Herstellung der Verbindungen der Formel II ist weiter oben bereits illustriert worden.

Die Verbindungen der Formel III können etwa aus den entsprechenden Benzoesäuren, Benzoylchloriden oder Benzoesäureestern bzw. Benzaldehyden durch Umsetzung mit Ammoniak bzw. Hydroxylamin erhalten werden. Die benötigten Benzaldehyde sind beispielsweise durch kontrollierte Oxydation des entsprechenden Benzylalkohols oder durch Formylierung von entsprechendem Phenol und nachfolgende Veretherung herstellbar.

Die erfindungsgemässen Dielektrika bestehen aus 2 bis 15, vorzugsweise 3 bis 12 Komponenten, darunter mindestens einem Benzonitril der Formel I. Die anderen Bestandteile werden vorzugsweise ausgewählt aus den nematischen oder nematogenen Substanzen, insbesondere den bekannten Substanzen, aus den Klassen der Azoxybenzole, Benzylidenaniline, Biphenyle, Terphenyle, Phenyl- oder Cyclohexylbenzoate, Cyclohexancarbonsäurephenyl- oder -cyclohexyl-ester, Phenylcyclohexane, Cyclohexylbiphenyle, Cyclohexylcyclohexane, Cyclohexylnaphthaline, 1,4-Bis-cyclohexylbenzole, 4,4'-Bis-cyclohexylbiphenyle, Phenyl- oder Cyclohexylpyrimidine, Phenyl- oder Cyclohexyldioxane, gegebenenfalls halogenierten Stilbene, Benzylphenylether, Tolane und substituierten Zimtsäuren.

Die wichtigsten als Bestandteile derartiger flüssigkristalliner Dielektrika in Frage kommenden Verbindungen lassen sich durch die Formel IV charakterisieren,

$R^5$-A-B-D-$R^6$ IV

worin A und D je ein carbo- oder heterocyclisches Ringsystem aus der aus 1,4-disubstituierten Benzol- und Cyclohexanringen, 4,4'-disubstituierten Biphenyl-, Phenylcyclohexan- und Cyclohexylcyclohexansystemen, 2,5-disubstituierten Pyrimidin- und 1,3-Dioxanringen, 2,6-disubstituiertem Naphthalin, Di- und Tetrahydronaphthalin, Chinazolin und Tetrahydrochinazolin gebildeten Gruppe,

| | | |
|---|---|---|
| B | -CH=CH- | -N(O)=N- |
| | -CH=CY- | -CH=N(O)- |
| | -C≡C- | $-CH_2-CH_2-$ |
| | -CO-O- | $-CH_2-O-$ |
| | -CO-S- | $-CH_2-S-$ |
| | -CH=N- | -COO-Ph-COO- |

oder eine C-C-Einfachbindung, Y Halogen, vorzugsweise Chlor, oder -CN, und $R^5$ und $R^6$ Alkyl, Alkoxy, Alkanoyloxy oder Alkoxycarbonyloxy mit bis zu 18, vorzugsweise bis zu 8 Kohlenstoffatomen, oder einer dieser Reste auch -CN, -NC, $-NO_2$, $-CF_3$, F, Cl oder Br bedeuten.

Bei den meisten dieser Verbindungen sind $R^5$ und $R^6$ voneinander verschieden, wobei einer dieser Reste meist eine Alkyl- oder Alkoxygruppe ist. Aber auch andere Varianten der vorgesehenen Substituenten sind gebräuchlich. Viele solcher Substanzen oder auch Gemische davon sind im Handel erhältlich.

Die erfindungsgemässen Dielektrika enthalten von 0.1 bis etwa 60 Gew% der Verbindungen der Formel I. In der Regel enthalten sie 5 bis 55 %, vorzugsweise 10 bis 50 Gew% einer oder mehrerer Verbindungen der Formel I.

Die Herstellung der erfindungsgemässen Dielektrika erfolgt in an sich üblicher Weise. In der Regel wird die gewünschte Menge der in geringerer Menge verwendeten Komponenten in der den Hauptbestandteil ausmachenden Komponente gelöst, zweckmässig bei erhöhter Temperatur. Wenn dabei eine Temperatur oberhalb des Klärpunkts des Hauptbestandteils gewählt wird, kann die Vollständigkeit des Lösevorgangs besonders leicht beobachtet werden.

Durch geeignete Zusätze können die flüssigkristallinen Dielektrika nach der Erfindung so modifiziert werden, dass sie in allen bisher bekannt gewordenen Arten von Flüssigkristallanzeigeelementen verwendet werden können.

Derartige Zusätze sind dem Fachmann geläufig und in der einschlägigen Literatur ausführlich beschrieben. Beispielsweise können Leitsalze, vorzugsweise Ethyl-dimethyl-dodecyl-ammonium-4-hexyloxybenzoat, Tetrabutylammonium-tetraphenylboranat oder Komplexsalze von Kronenethern (vergl. z.B. I. Haller et al., Mol. Cryst. Liq. Cryst. Band 24, Seiten 249 - 258 (1973)) zur Verbesserung der Leitfähigkeit, dichroitische Farbstoffe oder Substanzen zur Veränderung der dielektrischen Anisotropie, der Viskosität und/oder der Orientierung der nematischen Phasen zugesetzt werden. Derartige Substanzen sind zum Beispiel in den DE-OS 22 09 127, 22 40 864, 23 21 632, 23 38 281, 24 50 088, 26 37 430, 28 53 728 und 29 02 177 beschrieben.

Die folgenden Beispiele sollen die Erfindung erläutern. In den Beispielen bedeuten F. den Schmelzpunkt und K. den Klärpunkt einer flüssigkristallinen Substanz in Grad Celsius.

**Beispiel 1**

Ein Gemisch von 375 g 3-Brom-4-butoxy-4'-pentyl-biphenyl [Öl; erhältlich durch Bromierung von 4-Butoxy-4'-pentylbiphenyl], 160 g $Cu_2(CN)_2$ und 1 1 N-Methylpyrrolidon wird 4 Stunden bei 175° gerührt, auf 20° abgekühlt und nach Stehen über Nacht in eine Lösung von 1 kg NaCN in 20 l Wasser gegossen. Nach einstündigem Rühren bei 95° wird mit Toluol extrahiert. Nach üblicher Aufarbeitung erhält man 3-Cyan-4-butoxy-4'-pentyl-biphenyl, F. 35°, K. -40°.

**Beispiele 2 bis 25**

Analog Beispiel 1 kann man durch Bromierung der entsprechenden Benzolderivate und anschließende Reaktion mit $Cu_2(CN)_2$ erhalten:

2. 4'-Propyl-4-methoxy-3-cyan-biphenyl.
3. 4'-Butyl-4-ethoxy-3-cyan-biphenyl.
4. 4'-Hexyl-4-ethoxy-3-cyan-biphenyl.
5. 4'-Octyl-4-propoxy-3-cyan-biphenyl.
6. 4'-Propyl-4-propoxy-3-cyan-biphenyl.
7. 4'-Butyl-4-propoxy-3-cyan-biphenyl.
8. 4'-Pentyl-4-butoxy-3-cyan-biphenyl.
9. 4'-Pentyl-4-hexoxy-3-cyan-biphenyl, F. 41°, K. 28°.
10. 4'-Hexyl-4-hexoxy-3-cyan-biphenyl.
11. 4'-(4-Propyl-bicyclo[2,2,2]octyl)-4-propoxy-3-cyan-biphenyl.
12. 4'-(4-Propyl-bicyclo[2,2,2]octyl)-4-methoxy-3-cyan-biphenyl.
13. 4'-(4-Butyl-bicyclo[2,2,2]octyl)-4-(2-methyl-butoxy)-3-cyan-biphenyl.
14. 4'-(4-Propyl-bicyclo[2,2,2]octyl)-4-hexoxy-3-cyan-biphenyl.
15. 2-Propoxy-5-(4-ethyl-bicyclo[2,2,2]octyl)-benzonitril.
16. 2-Butoxy-5-(4-ethyl-bicyclo[2,2,2]octyl)-benzonitril.
17. 2-Pentoxy-5-(4-ethyl-bicyclo[2,2,2]octyl)-benzonitril.
18. 2-Hexoxy-5-(4-ethyl-bicyclo[2,2,2]octyl)-benzonitril.
19. 2-Octoxy-5-(4-ethyl-bicyclo[2,2,2]octyl)-benzonitril.
20. 2-Propoxy-5-(4-(4'-propyl-biphenylyl))-benzonitril.
21. 2-Hexoxy-5-(4-(4'-butyl-biphenylyl))-benzonitril der Formel Butyl-Phe-Phe-Phe(CN)-O-Hexyl.
22. 2-Butoxy-5-(4-(4-propyl-phenyl)-bicyclo[2,2,2]octyl)-benzonitril der Formel Propyl-Phe-Bi-Phe(CN)-O-Butyl.
23. 2-Pentoxy-5-(4-(4-butyl-phenyl)-bicyclo[2,2,2]octyl)-benzonitril.
24. 2-Butoxy-5-(4'-propyl-4,4'-bis-(bicyclo[2,2,2]octyl))-benzonitril der Formel Propyl-Bi-Bi-Phe(CN)-O-Butyl.
25. 2-Hexoxy-5-(4'-pentyl-4,4'-bis-(bicyclo[2,2,2]octyl))-benzonitril.

**Beispiel 26**

Man löst 31,1 g 3-Amino-4-butoxy-4'-pentyl-biphenyl (erhältlich beispielsweise durch Nitrierung von 4-Butoxy-4'-pentyl-biphenyl und anschließend Hydrierung des erhaltenen 3-Nitro-4-butoxy-4'-pentyl-biphenyls) in einem Gemisch von 25 g konzentrierter Salzsäure und 75 ml Wasser und diazotiert bei 3 bis 6° mit einer Lösung von 8 g $NaNO_2$ in 15 ml Wasser.

Die Diazoniumsalzlösung wird einer auf 60 bis 70° erwärmten $Cu_2(CN)_2$-Lösung (hergestellt durch Erwärmen von 25 g Kupfersulfat mit 28 g KCN in 100 ml Wasser) innerhalb 15 Minuten zugesetzt. Man erwärmt noch 20 Minuten auf 100°, kühlt ab und erhält nach üblicher Aufarbeitung 3-Cyan-4-butoxy-4'-pentyl-biphenyl, F. 35°, K. -40°.

Analog sind aus den entsprechenden Aromaten die in den Beispielen 2 bis 25 aufgeführten Benzonitril-Derivate erhältlich.

**Beispiel 27**

Man versetzt eine Suspension von 33,9 g 3-Carbamoyl-4-butoxy-4'-pentyl-biphenyl (erhältlich beispielsweise durch Umsetzung von 4-Hydroxy-4'-pentyl-biphenyl mit Chloroform/KOH zu 3-Formyl-4-hydroxy-4'-pentyl-biphenyl, Butylierung zur 4-Butoxyverbindung, Oxydation zur Säure, Umsetzung mit $SOCl_2$ zum Chlorid und Reaktion mit $NH_3$) in 100 ml Toluol bei 80° mit 18 g $SOCl_2$. Nach 6stündigem Rühren bei 80°, Abkühlen und Gießen in Wasser erhält man 3-Cyan-4-butoxy-4'-pentyl-biphenyl, F. 35°, K. -40°.

Analog sind auch die in den Beispielen 2 bis 25 aufgeführten Benzonitril-Derivate erhältlich.

**Beispiel 28**

Man erhitzt ein Gemisch aus 33,9 g 2-Butoxy-5-p-pentyl-phenyl-benzaldoxim (erhältlich aus dem Aldehyd und Hydroxylamin) mit 40 ml Acetanhydrid bis zum Beginn der exothermen Reaktion, kocht anschließend 20 Minuten, gießt in Wasser und erhält 3-Cyan-4-butoxy-4'-pentyl-biphenyl, F. 35°, K. -40°.

Analog sind auch die in den Beispielen 2 bis 25 aufgeführten Benzonitril-Derivate erhältlich.

Durch Entwässerung entsprechender Oxime kann man außerdem erhalten:

29. 4,4'-Dibutyl-2-cyan-biphenyl.
30. 4,4'-Dihexyl-2-cyan-biphenyl.
31. 2-Pentoxy-6-(4-butyl-bicyclo[2,2,2]octyl)-benzonitril.
32. 2-Pentoxy-6-(4-(4'-propyl-biphenylyl))-benzonitril.

Die folgenden Beispiele sind solche für erfindungsgemäße Dielektrika mit einem Gehalt an mindestens einer Verbindung der Formel 1.

**Beispiel A**

Eine Mischung aus
5 % trans-1-p-Ethoxyphenyl-4-propyl-cyclohexan
8 % 1-p-(trans-4-Pentylcyclohexyl)-phenyl-pentan-1,3-dion
13 % 3-Cyan-4-butoxy-4'-pentyl-biphenyl
30 % 4-Ethyl-2'-fluor-4'-(trans-4-Pentylcyclohexyl)-biphenyl
23 % p-Methoxybenzoesäure-(p-Pentylphenylester)
8 % 4-Ethyl-4'-(trans-4-propylcyclohexyl)-biphenyl
7 % 4-Ethyl-4'-(trans-4-Pentylcyclohexyl)-biphenyl
6 % 4-(trans-4-Pentylcyclohexyl)-4'-(trans-4-propyl-cyclohexyl)-biphenyl
zeigt folgende Eigenschaften: F. -14°, K. 83°.

**Beispiel B**

Eine Mischung aus
23 % trans-1-p-Ethylphenyl-4-propyl-cyclohexan
15 % 3-Cyan-4-ethoxy-4'-propyl-biphenyl
26 % 4-Ethyl-2'-fluor-4'-(trans-4-Pentylcyclohexyl)-biphenyl
16 % 4-Ethyl-4'-(trans-4-propylcyclohexyl)-biphenyl
10 % 4-Ethyl-4'-(trans-4-Pentylcyclohexyl)-biphenyl
10 % 4-(trans-4-Pentylcyclohexyl)-4'-(trans-4-propyl-cyclohexyl)-biphenyl
zeigt folgende Eigenschaften: F. -13°, K. 67°.

**Patentansprüche**

1. Benzonitrile der Formel I

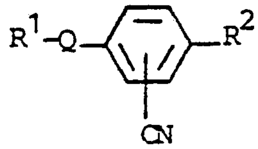

I

worin
$R^1$ und $R^2$ jeweils Alkylgruppen mit 1 - 8 C-Atomen, einer dieser Reste auch eine Alkoxygruppe mit 1 - 8 C-Atomen und
Q einen oder zwei Reste, ausgewählt aus der Gruppe bestehend aus 1,4-Phenylen und/oder 1,4-Bicyclo[2,2,2]-octylen
bedeuten.

2. Verfahren zur Herstellung von Benzonitrilen der Formel I

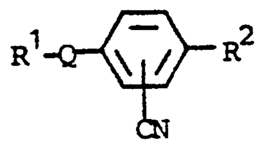

I

worin
$R^1$ und $R^2$ jeweils Alkylgruppen mit 1 - 8 C-Atomen, einer dieser Reste auch eine Alkoxygruppe mit 1 - 8 C-Atomen und
Q einen oder zwei Reste, ausgewählt aus der Gruppe bestehend aus 1,4-Phenylen und/oder 1,4-Bicyclo[2,2,2]-octylen
bedeuten,
dadurch gekennzeichnet, daß man eine Verbindung der Formel II

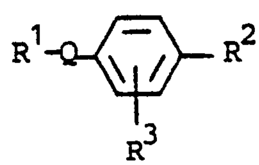

II

worin
$R^3$ Cl, Br, J oder eine Diazoniumsalzgruppe bedeutet und
$R^1$ und $R^2$ die angegebene Bedeutung haben mit einem Metallcyanid umsetzt
oder daß man eine Verbindung der Formel III

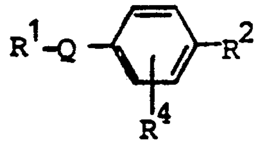

III

worin
$R^4$ -$CONH_2$ oder -CH=NOH
bedeutet und
$R^1$ und $R^2$ die angegebene Bedeutung haben dehydratisiert.

3. Verwendung der Benzonitrile der Formel I nach Anspruch 1 als Komponenten flüssigkristalliner Dielektrika für elektrooptische Anzeigeelemente.

4. Flüssigkristallines Dielektrikum für elektrooptische Anzeigeelemente mit mindestens zwei flüssigkristallinen Komponenten, dadurch gekennzeichnet, daß mindestens eine Komponente ein Benzonitril der Formel I nach Anspruch 1 ist.

5. Elektrooptisches Anzeigeelement auf der Basis einer Flüssigkristallzelle, dadurch gekennzeichnet, daß die Flüssigkristallzelle ein Dielektrikum nach Anspruch 4 enthält.

## Claims

1. Benzonitriles of the formula I

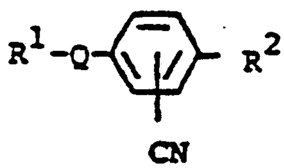

I

wherein $R^1$ and $R^2$ are each alkyl groups with 1 - 8 C atoms, and one of these radicals can also be an alkoxy group with 1 - 8 C atoms and Q is one or two radicals chosen from the group consisting of 1,4-phenylene and/or 1,4-bicyclo[2.2.2]-octylene.

2. Process for the preparation of benzonitriles of the formula I

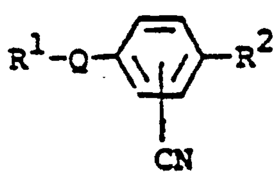

I

wherein $R^1$ and $R^2$ are each alkyl groups with 1 - 8 C atoms, and one of these radicals can also be an alkoxy group with 1 - 8 C atoms and Q is one or two radicals chosen from the group consisting of 1,4-phenylene and/or 1,4-bicyclo[2.2.2]-octylene, characterised in that a compound of the formula II

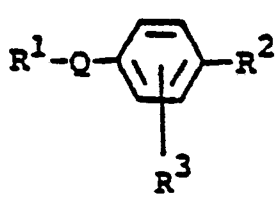

II

wherein $R^3$ is Cl, Br, I or a diazonium salt group and $R^1$ and $R^2$ have the meanings given, is reacted with a metal cyanide, or in that a compound of the formula III

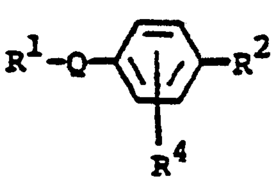

III

wherein $R^4$ is $-CONH_2$ or $-CH=NOH$ and $R^1$ and $R^2$ have the meanings given, is dehydrated.

3. Use of the benzonitriles of the formula I according to Claim 1 as components of liquid crystal dielectrics for electrooptical display elements.

4. Liquid crystal dielectric for electrooptical display elements with at least two liquid crystal components, characterised in that at least one component is a benzonitrile of the formula I according to Claim 1.

5. Electrooptical display element based on a liquid crystal cell, characterised in that the liquid crystal cell contains a dielectric according to Claim 4.

**Revendications**

1- Benzonitriles de formule I

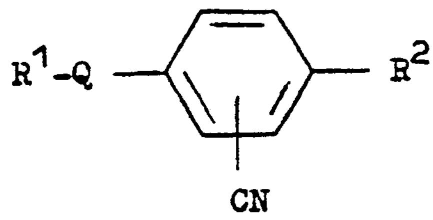

I

dans laquelle

$R^1$ et $R^2$ représentent chacun un groupe alkyle en C 1-C 8, l'un de ces symboles pouvant également representer un groupe alcoxy en C 1-C 8, et

Q représente un ou deux restes choisis dans le groupe consistant en les restes 1,4-phénylène et/ou 1,4-bicyclo[2,2,2]octylène.

2 - Procédé de préparation des benzonitriles de formule I

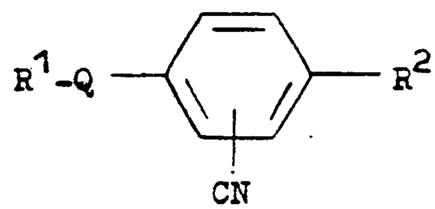

I

dans laquelle

$R^1$ et $R^2$ représentent chacun un groupe alkyle en C 1-C 8, l'un de ces symboles pouvant également représenter un groupe alcoxy en C 1-C 8, et

Q représente un ou deux restes choisis dans le groupe consistant en les restes 1,4-phénylène et/ou 1,4-bicyclo[2,2,2]octylène,

caractérisè en ce que l'on fait réagir un composé de formule II

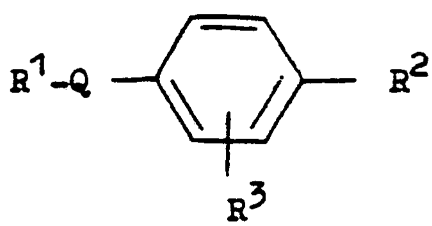

II

dans laquelle

$R^3$ représente Cl, Br, I ou un groupe sel de diazonium, et

$R^1$ et $R^2$ ont les significations indiquées ci-dessus, avec un cyanure métallique, ou bien on déshydrate un composé de formule III

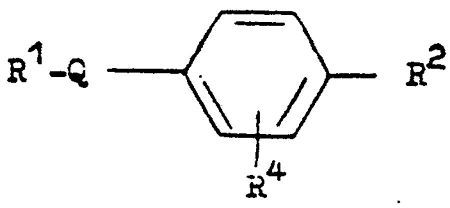

III

dans laquelle
$R^4$ représente $-CON_2$ ou $-CH=NOH$, et
$R^1$ et $R^2$ ont les significations indiquées ci-dessus.

3 - Utilisation des benzonitriles de formule I selon la revendication 1 en tant que composants de diélectriques à cristaux liquides pour éléments d'affichage électro-optiques.

4 - Diélectrique à cristaux liquides pour éléments d'affichage électro-optiques, contenant au moins deux composants à cristaux liquides, caractérisé en ce que l'un au moins des composants est un benzonitrile de formule I selon la revendication 1.

5 - Elément d'affichage électro-optique à base d'une cellule à cristaux liquides, caractérisé en ce que la cellule à cristaux liquides contient un diélectrique selon la revendication 4.